(19) Europäisches Patentamt
European Patent Office
Office européen des brevets

(11) EP 1 371 700 A2

(12) # EUROPEAN PATENT APPLICATION

(43) Date of publication:
**17.12.2003 Bulletin 2003/51**

(21) Application number: **03253679.9**

(22) Date of filing: **11.06.2003**

(51) Int Cl.[7]: **C09J 4/06**, C09D 4/06, C08F 265/06, C09J 5/00, C09J 5/06, C08J 7/04, C09D 7/12, C08F 222/10

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **14.06.2002 US 389043 P**
**30.09.2002 US 414588 P**

(71) Applicant: **ROHM AND HAAS COMPANY**
**Philadelphia, Pennsylvania 19106-2399 (US)**

(72) Inventors:
- **Kauffman, Thomas Frederick**
**Harleysville, Pennsylvania 19438 (US)**
- **Whitman, David W.**
**Harleysville, Pennsylvania 19438 (US)**

(74) Representative: **Kent, Venetia Katherine**
**Rohm and Haas (UK) Ltd**
**European Operations Patent Dept.**
**Lennig House**
**2 Mason's Avenue**
**Croydon, CR9 3NB (GB)**

(54) **Curable fluids for forming coatings and adhesives**

(57) Provided is a curable fluid with relatively low viscosity capable of forming a polymeric composition, subsequent to exposure to cure conditions. Also provided are coated articles made of substrates coated by such compositions, and further provided are composite articles made of substrates bonded by such compositions; further provided are methods of making such coated and composite articles.

EP 1 371 700 A2

## Description

## BACKGROUND:

This invention relates to a method of preparing a coated or composite article, to a coated or composite article formed thereby, and to a curable fluid useful for forming such coated or composite articles. In particular, the method of this invention includes forming an assembly containing a substrate and a curable fluid containing polymeric nanoparticles. Additionally, the method includes exposing the curable fluid to cure conditions, either before or after the formation of the assembly, to form a polymeric composition that functions as a coating or as an adhesive. Desirable are fluids with low enough viscosity to be easily applied to a substrate. Coated articles are prepared by forming a polymeric coating composition on a surface of a substrate, where some or all of the polymeric coating composition is not in contact with any other substrate. Composite articles are prepared by bonding two or more substrates with a polymeric adhesive composition. The polymeric composition desirably has good adhesion to the substrate(s).

Some composite articles are made of relatively thin, flat layers; such composite articles are generally known as laminates. The method of this invention is useful for preparing various types of composite articles, including laminates, especially flexible laminates. Laminates are used to provide packaging which is light-weight and flexible. Typically, laminates are formed from combinations of various polymeric films, including, for example, polymeric substrates with low surface energies, bonded by a bonding composition to identical polymeric films, to different films, and/or to metal foils. It is desirable to use the bonding composition at a low application weight to minimize the weight of the laminate, to maintain flexibility, and to minimize cost.

New coating and bonding methods are desired which allow the preparation of coated or composite articles, including those made from opaque substrates. Multilayered composite articles are also desired which may be formed with a single cure step. One approach to these goals has been to apply a curable liquid and then expose that curable liquid to elevated temperature or to radiation such as, for example, ultraviolet (UV) radiation or electron beam (E-beam) radiation. Cure with UV radiation often requires the use of photoinitiators, and cure with E-beam radiation generally does not require the use of photoinitiators.

Adhesives cured by radiation have been disclosed by US Patent Application 2002/0016381, which describes adhesives containing vinyl modified block copolymers. However, vinyl modified block copolymers are uncommon materials, which are potentially expensive and potentially difficult to make and/or obtain. Furthermore, formulations containing vinyl modified block copolymers sometimes have very high viscosities, making the formulation difficult to coat onto a substrate. A class of ingredients that is useful in making curable adhesives is the class of polymeric nanoparticles (PNPs); some methods of making and using PNPs are disclosed in US Patent Application 10/097256. An object of the present invention is to provide curable fluids that, prior to cure, have desirably low viscosity and that, after exposure to cure conditions, have desirably high adhesion to substrate(s).

## STATEMENT OF THE INVENTION:

In a first aspect of the present invention, there is provided a curable fluid comprising polymeric nanoparticles, wherein said curable fluid is capable of forming a polymeric composition after exposure to at least one cure condition selected from the group consisting of elevated temperature, radiation, mixing of moieties with complementary reactive groups, and combinations thereof; wherein said polymeric nanoparticles comprise, as polymerized units, at least one multiethylenically unsaturated monomer; and wherein said polymeric nanoparticles have mean diameter of 1 nm to 50 nm.

In a second aspect of the present invention, there is provided a composite article comprising a first substrate, a polymeric adhesive composition, and at least one subsequent substrate, wherein said composite article is formed by a process comprising:

(a) applying a layer of a curable fluid comprising polymeric nanoparticles, wherein said polymeric nanoparticles comprise, as polymerized units, at least one multiethylenically unsaturated monomer, and wherein said polymeric nanoparticles have mean diameter of 1 nm to 50 nm,
(b) contacting said layer to said subsequent substrate, and
(c) exposing said curable fluid to at least one cure condition selected from the group consisting of elevated temperature, radiation, mixing of moieties with complementary reactive groups, and combinations thereof.

In a third aspect of the present invention, there is provided a method for forming a composite article comprising

(a) applying a layer of a curable fluid comprising polymeric nanoparticles to a first substrate, wherein said polymeric nanoparticles comprise, as polymerized units, at least one multiethylenically unsaturated monomer, and wherein

said polymeric nanoparticles have mean diameter of 1 nm to 50 nm,
(b) contacting said layer to at least one subsequent substrate, and
(c) exposing said curable fluid to at least one cure condition selected from the group consisting of elevated temperature, radiation, mixing of moieties with complementary reactive groups, and combinations thereof;

wherein, subsequent to exposure of said curable fluid to said cure condition, a polymeric adhesive composition has formed.

In a fourth aspect of the present invention, there is provided a coated article comprising a substrate and a polymeric coating composition, wherein said coated article is formed by a process comprising:

(a) applying a layer of a curable fluid comprising polymeric nanoparticles to said substrate, wherein said polymeric nanoparticles comprise, as polymerized units, at least one multiethylenically unsaturated monomer, and wherein said polymeric nanoparticles have mean diameter of 1 nm to 50 nm, and
(b) exposing said curable fluid to at least one cure condition selected from the group consisting of elevated temperature, radiation, mixing of moieties with complementary reactive groups, and combinations thereof.

In a fifth aspect of the present invention, there is provided a method for forming a coated article comprising

(a) applying a layer of a curable fluid comprising polymeric nanoparticles to a substrate, wherein said polymeric nanoparticles comprise, as polymerized units, at least one multiethylenically unsaturated monomer, and wherein said polymeric nanoparticles have mean diameter of 1 nm to 50 nm, and
(b) exposing said curable fluid to at least one cure condition selected from the group consisting of elevated temperature, radiation, mixing of moieties with complementary reactive groups, and combinations thereof;

wherein, subsequent to exposure of said curable fluid to said cure condition, a polymeric coating composition has formed.

## DETAILED DESCRIPTION

The practice of the present invention involves the use of a curable fluid that contains polymeric nanoparticles. The curable fluid optionally also contains one or more polymerizable compounds, and the curable fluid may optionally further contain other ingredients. The curable fluid may be contacted with one or more substrates to form an assembly; it is contemplated that, if the curable fluid is not exposed to cure conditions, the assembly would have relatively poor adhesion (i. e., if the attempt were made to separate the substrate from the curable fluid, separation could be accomplished with minimal force). However, some time after the curable fluid is exposed to one or more cure conditions, some or all of the curable fluid will have formed a polymeric composition, and the assembly will have become a coated article or a composite article, which will have good adhesion (i.e., significant force would be required to separate the substrate and the polymeric composition).

As used herein, the term "dispersion" refers to a physical state of matter that includes at least two distinct phases wherein a first phase is distributed in a second phase, the second phase being a continuous medium. By "aqueous" medium is meant herein a medium that is from 50 weight% to 100 weight% water, based on the weight of the aqueous medium. A "nonaqueous" medium is meant herein a medium that is from 0 weight% to less than 50 weight% water, based on the weight of the nonaqueous medium.

The term "(meth)acrylic" used herein includes both acrylic and methacrylic and the term "(meth)acrylate" includes both acrylate and methacrylate. Likewise, the term "(meth)acrylamide" refers to both acrylamide and methacrylamide. "Alkyl" includes straight chain, branched and cyclic alkyl groups.

The practice of the present invention includes the use of a curable fluid. By "fluid" is meant a liquid with viscosity of 10 Pa•s (10,000 cps) or less at 60°C, as measured by standard methods, for example using Brookfield viscometer model DVI with a #25 spindle. Liquids with viscosity of 10 Pa•s (10,000 cps) or less at lower temperatures (for example, 20°C) are assumed to have even lower viscosity at 60°C than they have at such lower temperature, and so they are considered as "fluid" herein.

A "curable" moiety as used herein is a moiety that contains chemical reactivity sufficient to form, upon exposure to one or more cure conditions, chemical attachment to at least one other moiety. The curable moiety forms a chemical attachment to a moiety that is the same or different. For example, the curable moiety may form a chemical attachment to a polymeric nanoparticle, to a substrate, to other ingredients of the curable fluid, or to the polymeric composition. Chemical attachment herein means that the curable moiety and the other moiety can be re-separated only by further chemical reaction and not by physical means such as dissolution in solvent. In many embodiments, curable moieties will be curable because they contain functional groups that are capable of reacting, upon exposure to cure conditions,

with identical, similar, or complementary (as defined herein below) reactive groups.

In the practice of the present invention, the curable fluid is exposed to one or more cure conditions, and a polymeric composition is formed. The polymeric composition of the present invention is useful in some embodiments as a polymeric coating composition and in some embodiments as a polymeric adhesive composition.

The practice of the present invention includes the use of polymeric particles having a mean diameter in the range of from 1 nanometer (nm) to 50 nm, the particles including, as polymerized units, at least one multiethylenically unsaturated monomer. The polymeric particles, referred to herein as polymeric nanoparticles ("PNPs"), are addition polymers, which contain, as polymerized units, at least one multiethylenically unsaturated monomer. Suitable multiethylenically unsaturated monomers useful in the present invention include di-, tri-, tetra-, or higher multifunctional ethylenically unsaturated monomers such as, for example, divinyl benzene, trivinylbenzene, divinyltoluene, divinylpyridine, divinylnaphthalene, divinylxylene, ethyleneglycol di(meth)acrylate, trimethylolpropane tri(meth)acrylate, diethyleneglycol divinyl ether, trivinylcyclohexane, allyl (meth)acrylate, diethyleneglycol di(meth)acrylate, propyleneglycol di(meth)acrylate, trimethylolpropane tri(meth)acrylate, 2,2-dimethylpropane-1,3-di(meth)acrylate, 1,3-butylene glycol di(meth)acrylate, 1,4-butanediol di(meth)acrylate, 1,6-hexanediol di(meth)acrylate, tripropylene glycol di(meth)acrylate, triethylene glycol di(meth)acrylate, polyethylene glycol di(meth)acrylates, such as polyethylene glycol 200 di (meth)acrylate and polyethylene glycol 600 di(meth)acrylate, tetraethylene glycol di(meth)acrylate, ethoxylated bisphenol A di(meth)acrylate, poly(butanediol) di(meth)acrylate, pentaerythritol tri(meth)acrylate, trimethylolpropane triethoxy tri(meth)acrylate, glyceryl propoxy tri(meth)acrylate, pentaerythritol tetra(meth)acrylate, dipentaerythritol monohydroxypenta(meth)acrylate, divinyl silane, trivinyl silane, dimethyl divinyl silane, divinyl methyl silane, methyl trivinyl silane, diphenyl divinyl silane, divinyl phenyl silane, trivinyl phenyl silane, divinyl methyl phenyl silane, tetravinyl silane, dimethyl vinyl disiloxane, poly(methyl vinyl siloxane), poly(vinyl hydro siloxane), poly(phenyl vinyl siloxane), and mixtures thereof.

Typically, the PNPs contain at least 1% by weight based on the weight of the PNPs, of at least one polymerized multiethylenically unsaturated monomer. Up to and including 100% polymerized multiethylenically unsaturated monomer, based on the weight of the PNPs, can be effectively used in the particles of the present invention. It is preferred that the amount of polymerized multiethylenically unsaturated monomer is from 1% to 80%, more preferably from 1% to 60%, most preferably from 1% to 25%, by weight based on the weight of the PNPs.

The PNPs optionally contain as polymerized units one or more of a variety of other monomers that are not multiethylenically unsaturated monomers. Suitable amount of monomers that are not multiethylenically unsaturated in the PNPs, as polymerized units, is 0% to 99%, based on the weight of the PNPs; preferred is 20% to 99%; more preferred is 40% to 99%; most preferred is 75% to 99%. Some suitable other monomers include, for example, $C_1C_{24}$ alkyl (meth)acrylates such as methyl (meth)acrylate, ethyl (meth)acrylate, propyl (meth)acrylate, butyl (meth)acrylate, isobutyl (meth)acrylate, hexyl (meth)acrylate, cyclohexyl (meth)acrylate, 2-ethylhexyl (meth)acrylate, octyl (meth)acrylate, decyl (meth)acrylate, dodecyl (meth)acrylate, pentadecyl (meth)acrylate, hexadecyl (meth)acrylate, octadecyl (meth)acrylate, and nonadecyl (meth)acrylate, and mixtures thereof. Other suitable monomers include, for example, 2-hydroxyalkyl (meth)acrylates such as 2-hydroxyethyl (meth)acrylate, 2-hydroxypropyl (meth)acrylate, 1-methyl-2-hydroxyethyl (meth)acrylate, and 2-hydroxybutyl (meth)acrylate; ureido containing monomers such as N-(ethyleneureidoethyl) - 4-pentenamide, N-(ethylenethioureido-ethyl)-10-undecenamide, butyl ethyleneureido-ethyl fumarate, methyl ethyleneureido-ethyl fumarate, benzyl N-(ethyleneureido-ethyl) fumarate, and benzyl N-(ethyleneureido-ethyl) maleamate. Also suitable are vinyl acetate, vinyl versatate, and diisobutylene. Also suitable are vinylaromatic monomers such as styrene, $\alpha^-$ methylstyrene, vinyltoluene, p-methylstyrene, ethylvinylbenzene, vinylnaphthalene, vinylxylenes, and nonylphenoxy propenyl polyethoxylated alcohol. The vinylaromatic monomers also include their corresponding substituted counterparts, such as halogenated derivatives, i.e., containing one or more halogen groups, such as fluorine, chlorine or bromine; and nitro, cyano, $(C_1$-$C_{10})$alkoxy, halo$(C_1$-$C_{10})$alkyl, $(C_1$-$C_{10})$alkoxy, carboxy, and the like.

Also suitable for inclusion as polymerized units in some embodiments of the PNP of the present invention are substituted ethylene monomers including, for example, allylic monomers, vinyl acetate, vinyl formamide, vinyl chloride, vinyl fluoride, vinyl bromide, vinylidene chloride, vinylidene fluoride and vinylidene bromide.

In some embodiments, the PNPs contain, as polymerized units, at least one water soluble monomer. By "water soluble monomer" herein is meant a monomer having a solubility in water of at least 7 weight %, preferably at least 9 weight %, and most preferably as least 12 weight %, at a temperature of 25 °C. Data for the water solubility of monomers is found, for example, in "Polymer Handbook" (Second Edition, J. Brandrup, E.H. Immergut, Editors, John Wiley & Sons, New York) and "Merck Index"(Eleventh Edition, Merck & Co, Inc., Rahway, New Jersey). Examples of water soluble monomers include ethylenically unsaturated ionic monomers and ethylenically unsaturated water soluble nonionic monomers. The water soluble monomers, if used, may be either multiethylenically unsaturated or they may not be.

In some embodiments, the PNPs contain as polymerized units at least one ionic ethylenically unsaturated monomer, by which is meant herein that the monomer would bear an ionic charge if the PNPs were dispersed in an aqueous medium. The ionic ethylenically unsaturated monomer is referred to herein as "ionic monomer". The ionic

monomer may be multiethylenically unsaturated or it may not be. Suitable ionic monomers include, for example, acid-containing monomers, base-containing monomers; quaternized nitrogen-containing monomers, and other monomers that can be subsequently formed into ionic monomers such as monomers which can be neutralized by an acid-base reaction to form an ionic monomer. Suitable acid groups include carboxylic acid groups and strong acid groups such as phosphorus containing acids and sulfur containing acids. Suitable base groups include amines and amides.

In embodiments of the present invention in which the PNP includes acid-containing monomers as polymerized units, suitable acid-containing monomers include, for example, carboxylic acid monomers, such as (meth)acrylic acid, acryloxypropionic acid, and crotonic acid; dicarboxylic acid monomers such as itaconic acid, maleic acid, fumaric acid, and citraconic acid; and monomers with half esters of dicarboxylic acid groups such as monomers containing one carboxylic acid functionality and one $C_{1-6}$ ester. Preferred are acrylic acid, methacrylic acid, and mixtures thereof. Also suitable are strong acid monomers, which include, for example, sulfur acid monomers such as, for example, 2-acrylamido-2-methyl propane sulfonic acid, styrene sulfonic acid, styrene sulfinic acid, and vinyl sulfinic acid; and phosphorus acid monomers such as 2-phosphoethyl (meth)acrylate, vinyl phosphoric acid, vinyl phosphinic acid. Other suitable acid monomers include terminally unsaturated acid containing macromonomers as disclosed in U.S. Patent No. 5,710,227. Phosphorus acid monomers are desirable as they can provide improved adhesion to certain substrates (e.g., metal).

In embodiments of the present invention in which the PNP includes base-containing monomers as polymerized units, suitable base-containing monomers include, for example, monomers having amine functionality, which include N,N-dimethylaminoethyl (meth)acrylate, N,N-diethylaminoethyl (meth)acrylate, N-t-butylaminoethyl (meth)acrylate, N, N-dimethylaminopropyl (meth)acrylamide, p-aminostyrene, N,N-cyclohexylallylamine; allylamine, diallylamine, dimethylallylamine, N-ethyldimethylallylamine, crotyl amines, and N-ethylmethallylamine; monomers having pyridine functionality, which includes 2-vinylpyridine and 4-vinylpyridine; monomers having piperidine functionality, such as vinylpiperidines; and monomers having imidazole functionality, which includes vinyl imidazole. Other suitable base-containing monomers include oxazolidinylethyl (meth)acrylate, vinylbenzylamines, vinylphenylamines, substituted diallylamines, 2-morpholinoethyl (meth)acrylate, methacrylamidopropyl trimethyl ammonium chloride, diallyl dimethyl ammonium chloride, 2-trimethyl ammonium ethyl methacrylic chloride, and the like.

In some embodiments, the PNPs may contain as polymerized units at least one amphoteric monomer. Suitable amphoteric monomers include, for example, N-vinylimidazolium sulfonate inner salts and N,N-Dimethyl-N-(3-methacrylamidopropyl)-N-(3-sulfopropyl) ammonium betaine.

The PNPs of the present invention optionally contain functional groups, which have been provided by including, as polymerized units, monomers containing functional groups (also called "functional monomers" herein); functional groups introduced in this way are known herein as "primary" functional groups. Other functional groups can be optionally attached to the PNPs by taking PNPs with primary functional groups and reacting those primary functional groups with suitable modifying compounds, as taught in US 5,270,380. Generally, the reaction between the modifying compound and the primary functional groups will be non-radical. A suitable modifying compound is any compound that reacts usefully with the primary functional groups of the PNPs; however, modifying compounds are thought to be most useful when they are used to alter the functionality of the PNP. That is, most modifying compounds will have at least one "linking" functional group and at least one "secondary functional" group on the same molecule. Generally, the linking functional groups will react with the primary functional groups of the PNPs to form bonds between the modifying compounds and the PNPs; in this way, some or all of the primary functional groups of the PNPs will be converted to secondary functional groups. It is contemplated that secondary functional groups could be further modified by reaction with subsequent modifying compounds.

Various functional groups are suitable for use in the present invention. Any suitable functional group may be used as primary functional group, a linking functional group, and/or as a secondary functional group. Suitable functional groups include, for example, acetoacetate, aldehyde, amine or other base, anhydride, isocyanate, epoxy, hydrazide, carboxyl or other acid, carbodiimide, halide, chloro-methyl ester, chloromethyl amine, hydroxyl, aziridine, mercaptan, unsaturation, thiol, and mixtures thereof.

In the practice of the present invention, whenever one functional group can be reacted with a different functional group to form a useful bond, such a pair of functional groups is said herein to be "complementary." For example a hydroxyl functional group on first moiety may be made to react with a carboxyl functional group on a second moiety to form a bond (in this example, an ester linkage) between the moieties. Pairs of functional groups that are complementary include, for example: (a) acetoacetate-aldehyde; (b) acetoacetate-amine; (c) amine-aldehyde; (d) amine-anhydride; (e) amine-isocyanate; (f) amine-epoxy; (g) aldehyde-hydrazide; (h) acid-epoxy; (i) acid-carbodiimide; (j) acid-chloro methyl ester; (k) acid-chloro methyl amine; (l) acid-alcohol; (m) acid-anhydride; (n) acid-aziridine; (o) epoxy-mercaptan; and (p) isocyanate-alcohol.

In embodiments of the present invention that use modifying compounds, the reaction between the primary functional groups of the PNPs and the linking functional groups of the modifying compounds alternatively provide either ionic or covalent binding. Appropriate ionic binding includes acid-base interaction and ion pair binding of negatively

and positively charged atoms. Covalent binding may be provided by conducting a chemical reaction between complementary functional groups on the PNPs (*i.e.*, the "primary" functional groups) and on the modifying compounds (*i.e.,* the "linking" functional group). In any pair of complementary reactive groups, the first or second reactable group in each pair can be present in the PNPs or in the modifying compound.

In the practice of the present invention, an example of providing epoxy functionality as a primary functional group on PNPs would be PNPs made by including glycidyl (meth)acrylate and/or allyl glycidyl ether as polymerized units in the PNPs. Other monomers suitable for providing primary functionality include, for example, anhydride, such as maleic anhydride, an ester such as methyl acrylate, and halide-containing functional monomers. Suitable halide-containing functional monomers include, for example, vinylaromatic halides and halo-alkyl(meth)acrylates. Suitable vinylaromatic halides include vinylbenzyl chloride and vinylbenzyl bromide. Suitable halo-alkyl(meth)acrylates include chloromethyl (meth)acrylate. Other suitable functional monomers include allyl chloride, allyl bromide, and (meth)acrylic acid chloride.

In some embodiments, the PNPs contain as polymerized units at least one ethylenically unsaturated water soluble nonionic monomer (referred to herein as "water soluble nonionic monomer"). Examples of water soluble nonionic monomers include hydroxyalkyl (meth)acrylates such as hydroxyethyl (meth)acrylate and hydroxypropyl (meth) acrylate; poly(alkylene oxide) esters of (meth)acrylic acid such as poly(ethylene oxide)$_{20}$ methacrylate and poly(propylene oxide)$_{150}$ acrylate; acrylamide; and methacrylamide.

In the practice of the present invention, an example of providing unsaturation as a secondary functional group on PNPs would be PNPs made by including a hydroxyl-functional monomer such as, for example, hydroxyethyl methacrylate, as polymerized units in the PNPs; finished PNPs could then be reacted with acrylic acid. It is contemplated that the acid groups of acrylic acid will react with the hydroxyl groups of the PNPs, resulting in PNPs with acrylic functionality, including the unreacted double bond of the acrylic group, as secondary functionality of the PNPs.

In the practice of the present invention, one suitable method for providing PNPs is that of preparing a nonaqueous PNP dispersion containing the PNPs dispersed in at least one solvent.

A suitable polymerization process to prepare the nonaqueous PNP dispersion is free radical solution polymerization of at least one multiethylenically unsaturated monomer and, optionally, at least one other monomer. By "solution polymerization" herein is meant free radical addition polymerization in a suitable solvent for the polymer. By "suitable solvent for the polymer" herein is meant that linear random (co)-polymers having substantially similar polymerized monomer units to the PNPs, are soluble in the solvent. Another method for selecting a suitable solvent or mixture of solvents is on the basis of using solubility parameter analysis. According to such methods, the suitability of the solvent is determined by substantially matching the solubility parameters of the PNP and of the solvent, such as the Van Krevelen parameters of delta d, delta p, delta h and delta v. See, for example, Van Krevelen et al., Properties of Polymers. Their Estimation and Correlation with Chemical Structure, Elsevier Scientific Publishing Co., 1976; Olabisi et al., Polymer-Polymer Miscibility, Academic Press, NY, 1979; Coleman et al., Specific Interactions and the Miscibility of Polymer Blends, Technomic, 1991; and A. F. M. Barton, CRC Handbook of Solubility Parameters and Other Cohesion Parameters, 2$^{nd}$ Ed., CRC Press, 1991. Delta d is a measure of dispersive interactions, delta p is a measure of polar interactions, delta h is a measure of hydrogen bonding interactions, and delta v is a measure of both dispersive and polar interactions. Such solubility parameters are alternatively calculated, such as by the group contribution method, or determined experimentally as is known in the art. A preferred solvent has a delta v parameter within 5 (joule per cubic centimeter)½, preferably within 1 (joule per cubic centimeter)½ of the polymer delta v parameter. Suitable solvents for the polymerization include organic solvents such as hydrocarbons; alkanes; halohydrocarbons; chlorinated, fluorinated, and brominated hydrocarbons; aromatic hydrocarbons; ethers; ketones; esters; alcohols; and mixtures thereof. Particularly suitable solvents, depending on the composition of the PNP, include dodecane, mesitylene, xylenes, diphenyl ether, gamma-butyrolactone, ethyl acetate, ethyl lactate, propyleneglycol monomethyl ether acetate, caprolactone, 2-heptanone, methylisobutyl ketone, diisobutylketone, propyleneglycol monomethyl ether, and alkyl-alcohols, such as isopropanol, decanol, and t-butanol; and supercritical carbon dioxide.

In some embodiments, the nonaqueous PNP dispersion is prepared by first charging a solvent or, alternatively, a mixture of solvent and some portion of the monomers to a reaction vessel. The monomer charge is typically composed of monomers, an initiator, and a chain transfer agent. Typically, initiation temperatures are in the range of from 55 °C to about 125 °C, although lower or higher initiator temperatures are possible using suitable low temperature or high temperature initiators known in the art. After the heel charge has reached a temperature sufficient to initiate polymerization, the monomer charge or balance of the monomer charge is added to the reaction vessel. The monomer charge time period is typically in the range of from 15 minutes to 4 hours, although both shorter and longer time periods are envisioned. During the monomer charge, the reaction temperature is typically kept constant, although it is possible to vary the reaction temperature. After completing the monomer mixture addition, additional initiator in solvent can be charged to the reaction and/or the reaction mixture may be held for a time.

Control of PNP particle size and distribution can be achieved by one or more of such methods as choice of solvent, choice of initiator, total solids level, initiator level, type and amount of multi-functional monomer, type and

amount of ionic monomer, type and amount of chain transfer agent, and reaction conditions.

Initiators useful in the free radical polymerization of the present invention include, for example, one or more of: peroxyesters, alkylhydroperoxides, dialkylperoxides, azoinitiators, persulfates, redox initiators, and the like. The amount of the free radical initiator used is typically from 0.05% to 10% by weight, based on the weight of total monomer. Chain transfer reagents are optionally used to control the extent of the polymerization of the PNPs useful in the present invention. Suitable chain transfer agents include, for example: alkyl mercaptans such as dodecyl mercaptan, aromatic hydrocarbons with activated hydrogens such as toluene, and alkyl halides such as bromotrichloroethane.

The PNPs have a mean diameter in the range of from 1 nm to 50 nm, preferably in the range of from 1 nm to 40 nm, more preferably from 1 nm to 30 nm, even more preferably from 1 nm to 25 nm, even further preferably from 1 nm to 20 nm, and most preferably from 1 nm to 10 nm. It is further typical that the PNPs have a mean particle diameter of at least 1.5 nm, preferably at least 2 nm. One method of determining the particle sizes (mean particle diameter) of the PNPs is by using standard dynamic light scattering techniques, wherein the correlation functions can be converted to hydrodynamic sizes using LaPlace inversion methods, such as CONTIN.

Typically, PNPs including as polymerized units, less than 10 weight % multiethylenically unsaturated monomer, have a glass transition temperature from -90 °C to 170 °C for the composition in the absence of the polymerized multiethylenically unsaturated monomer, as determined by a modulated Differential Scanning Calorimetry measurement. PNPs containing as polymerized units, at least 50 weight % multiethylenically unsaturated monomer are considered to have glass transition temperatures of at least 50°C.The PNPs of the present invention typically have an "apparent weight average molecular weight" in the range of 5,000 to 1,000,000, preferably in the range of 10,000 to 500,000 and more preferably in the range of 15,000 to 100,000. As used herein, "apparent weight average molecular weight" reflects the size of the PNP particles using standard gel permeation chromatography methods, *e.g.*, using THF solvent at 40 °C, 3 Plgel™ Columns (Polymer Labs, Amherst, MA), 100 Angstrom (10 nm), $10^3$ Angstroms (100 nm), $10^4$ Angstroms (1 micron), 30 cm long, 7.8 mm ID, 1 milliliter per minute, 100 microliter injection volume, calibrated to narrow polystyrene standards using Polymer Labs CALIBRE ™ software.

The PNPs described above are ingredients in the curable fluid of the present invention, which is capable of forming a polymeric composition after exposure to at least one cure condition. Suitable weight percentages of the PNPs in the curable fluid of the present invention, based on total weight of the curable fluid, are typically from 1 weight% to 90 weight%, more typically from 2 weight% to 75 weight%, even more typically from 4 weight% to 65 weight%, further more typically from 8 weight% to 55 weight%, and most typically from 10 weight% to 45 weight%.

In some embodiments of the present invention, the PNPs are curable. In many embodiments, curable PNPs contain functional groups that are capable of reacting, upon exposure to cure conditions, with identical, similar, or complementary reactive groups. The functional groups attached to the PNPs may be primary or secondary functional groups.

The PNPs are desirably discrete or unagglomerated and dispersible, miscible or otherwise substantially compatible with/in the curable fluid and in the polymeric composition.

In some embodiments, the PNPs are used as a dispersion in the polymerization solvent. In some embodiments, the PNPs are isolated by, for example, vacuum evaporation, by precipitation into a non-solvent, and spray drying. When isolated, some PNPs can be subsequently redispersed in a medium appropriate for incorporation into a curable fluid. It is contemplated that, if isolation is desired, the method will be chosen according to the nature of the PNPs and of the curable fluid. In some cases isolation will be undesirable; for example, some PNPs with Tg above 20°C may yield freely flowing powders when isolated, which may be desirable in some embodiments, while some PNPs with Tg below 20°C will be tacky or otherwise difficult to handle when isolated.

The PNPs can be incorporated into a curable fluid by admixing the PNPs or a dispersion of the PNPs with other dissolved or dispersed polymers and/or other adjuvants as are well known to those skilled in the art. The curable fluid can include an aqueous or nonaqueous medium.

In some embodiments of the present invention, the curable fluid contains one or more polymerizable compounds. Suitable polymerizable compounds may be monomers, oligomers, resins, polymers, or mixtures thereof. Monomers are polymerizable compounds with relatively low molecular weight, usually 1,000 or less. Oligomers are linear, branched, or star-structured compounds of 2 to 10 monomer units; molecular weights of oligomers vary according to the molecular weight of the monomer units, but typical oligomers have molecular weights (Mn, or number-average molecular weight, as measured by gel permeation chromatography) of 10,000 or less. Polymers are linear, branched, comb-structured, star-structured, or crosslinked compounds of 11 or more monomer units, typically with Mn of more than 10,000. The term "resin" is used to mean either a polymer or an oligomer. For any oligomer, resin, or polymer to function as polymerizable compound, it must be capable of further polymerization during or after exposure to cure conditions. Each molecule of suitable polymerizable compound may have one or more reactive groups capable of participating in a polymerization reaction. Additionally, curable PNPs function as polymerizable compounds.

Preferred are polymerizable compounds with low volatility. Generally, compounds with relatively high boiling points are believed to have low volatility. Preferred as polymerizable compounds in the present invention are compounds with boiling point of 60°C or above; more preferably 80°C or above; even more preferably 100°C or above, and most preferably 120°C or above.

A preferred class of compounds suitable for use in the present invention as polymerizable compound are acrylic compounds, which are any compounds containing (meth)acrylic groups. Suitable acrylic compounds include, for example, (meth)acrylic acid, esters of (meth)acrylic acid, adducts of (meth)acrylic acid and/or (meth)acrylate esters with other functional compounds, and mixtures thereof. Among the esters of (meth)acrylic acid that are suitable for use as polymerizable compound are, for example, alkyl esters of (meth)acrylic acid; hydroxyl containing esters of (meth)acrylic acid such as for example hydroxyethyl (meth)acrylate; ring containing esters of (meth)acrylic acid such as for example isobornyl (meth)acrylate; esters of (meth)acrylic acid containing other groups such as for example ethylene oxide, allyl groups, glycidyl groups, and the like; and mixtures thereof.

Also among the acrylic compounds suitable for use in the present invention as polymerizable compound are acrylic compounds that have tertiary alkyl amide functionality. One group of suitable acrylic compounds that have tertiary alkyl amide functionality is the group of substituted (meth)acrylamides that have the following structure:

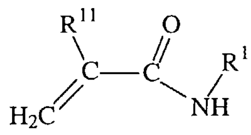

where $R^{11}$ is either hydrogen or methyl, and $R^1$ is

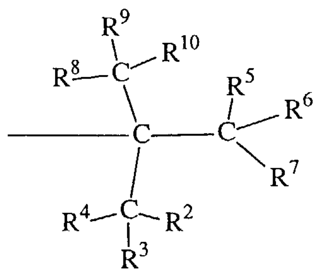

where $R^2$-$R^{10}$ are, independently, hydrogen or organic groups. The carbon atom of $R^1$ that is attached to the amide nitrogen is a tertiary alkyl carbon atom, so the functional group is said to have "tertiary-alkyl amide functionality." If any of $R^2$-$R^{10}$ are organic groups, they may be independently alkyl, cycloalkyl, aryl, alkylaryl, unsaturated, and/or substituted with one or more halogen, amide, sulfonic, carbonyl, or other groups. Preferred acrylic compounds that have tertiary-alkyl amide functionality are 2-acrylamido 2-methylpropane sulfonic acid, diacetone (meth)acrylamide, N-tert-butyl (meth)acrylamide, N-tert-octyl (meth)acrylamide, and mixtures thereof; more preferred are diacetone acrylamide, N-tert-butyl acrylamide, and mixtures thereof.

Additionally among the acrylic compounds suitable as polymerizable compound for use in the present invention are esters of (meth) acrylic acid or other substituted acrylic acid compounds with polyols, such as for example esters of (meth)acrylic acid with polyols, including esters of (meth)acrylic acid with alkoxylated polyols. Some suitable esters of (meth)acrylic acid with alkoxylated polyols include, for example, esters of (meth)acrylic acid with ethoxylated pentaerythritol, propoxylated pentaerythritol, ethoxylated trimethylolpropane, propoxylated trimethylolpropane, ethoxylated hexane diols, propoxylated hexane diols, similar polyols in which some hydroxyls are ethoxylated and other hydroxyls are propoxylated, and mixtures thereof.

Further additionally included in the class of acrylic compounds suitable for use as polymerizable compounds of the present invention are adducts of any of the above acrylic compounds with other functional compounds such as for example epoxy compounds, isocyanates, or phosphate compounds. Examples of adducts of acrylic compounds with other functional compounds that are suitable as a polymerizable compound include, for example, epoxy (meth) acrylates, the adducts of alkyl isocyanates with hydroxyalkyl (meth)acrylates, and (meth)acrylate-terminated phosphate esters. Some adducts known to be suitable as a polymerizable compound are, for example, Ebecryl™ CL 1039, a urethane monoacrylate monomer supplied by UCB chemicals; and Ebecryl™ 111, an epoxy monoacrylate monomer supplied by UCB chemicals.

Yet another group of acrylic compounds suitable for use in the present invention as polymerizable compounds are acrylic curable oligomers: that is, curable oligomers made fully or partially from (meth)acrylic monomers. Curable oligomers have a functional group such as a residual acrylic double bond or any of the other functional groups disclosed

herein above. Some suitable such curable oligomers may be made by reacting one or more acrylic monomers with each other to form an oligomer. Other suitable acrylic oligomers may be made by reacting one or more acrylic monomers with other compounds to form suitable oligomers. When oligomers are used, preferred are those obtained by reaction of one or more (meth)acrylic acid, (meth)acryloyl halide, and/or (meth)acrylate ester with one or more of hydroxy-containing alkyd resins, polyester condensates, or polyether condensates, as disclosed in US Patent Application Number 10/135258.

Compounds are suitable for use in the present invention because of their chemical structure, regardless of the method of synthesis or manufacture. Consequently, it is to be understood that, in the descriptions herein of chemical compounds, words like "esterified" and "adducts" and "ethoxylated" are used to describe chemical structures, regardless of the method of making those chemicals.

In addition to (meth)acrylate compounds, other polymerizable compounds are suitable for use in the present invention as polymerizable compound. Suitable compounds include for example ethylenically unsaturated compounds such as vinyl acetate, derivatives of vinyl acetate, substituted vinyl acetate compounds, styrene, substituted styrenes such as alpha-methyl styrene, and mixtures thereof. Also suitable are other compounds that are able to polymerize or copolymerize during or after exposure to cure conditions such as for example urethanes, epoxies, anhydrides; compounds capable of ring-opening polymerization, and mixtures thereof.

Another group of compounds suitable for use in the present invention as polymerizable compounds are polymers capable of further curing when exposed to cure conditions. One group of such polymers have one or two terminal ethylenically unsaturated groups; some water-insoluble examples of such polymers are disclosed in US Patent Application Number 09/951924.

Any mixtures of polymerizable compounds suitable for use in the present invention will also be suitable as polymerizable compound. Preferred polymerizable compounds are monomers, curable oligomers, curable PNPs, and mixtures thereof; more preferred are polymerizable compounds that contain only monomers, curable PNPs, curable oligomers, and mixtures thereof. Also preferred are polymerizable compounds that contain only acrylic compounds.

The curable fluid of the present invention may contain, in addition to PNPs, other compounds that are not polymerizable (*i.e.,* "non-polymerizable"). Examples of suitable additional non-polymerizable compounds include, for example, polymers; resins; diluents; solvents; tackifiers; pigments; emulsifiers; biocides; plasticizers; non-curable PNPs; waxes; coalescing agents; and additives that improve the flow of the composition, that help the composition wet the substrate, that reduce foaming, or that adjust the viscosity of the composition. Ingredients in the curable fluid may be present in solution, in dispersion, or in a mixture thereof. Preferred are curable fluids that use little or no solvent. Also generally preferred are curable fluids with high solids level. The solids level is the sum of the weights of all PNPs, all polymerizable compounds, all polymers and all remaining (*i.e.*, other than PNPs, polymers, and polymerizable compounds) solid ingredients, expressed as a percentage of the total weight of the curable fluid. Solid remaining ingredients are those that, in pure form, are solid at 25°C.

In the practice of the present invention, the curable fluid is applied onto a substrate. Application may be performed by any means, including for example, manual or mechanical spreading. Suitable application methods include for example roll coating, rod coating, gravure, Meyer bar, and the like. The curable fluid may be applied at room temperature (20°C to 25°C); it may be applied hot (*i.e.*, at a temperature above room temperature); or it may be applied cool (*i.e.,* at a temperature below room temperature). Typically, hot application is performed if it is desired to reduce the viscosity of the curable fluid to improve the operation of the application method. If hot application is used, the temperature is chosen to prevent or minimize polymerization during application of the curable fluid; if hot application is performed, it is generally performed at 70°C or lower. Typically, cool application is performed if it is desired to reduce the rate of the curing reaction; cool application is often used in embodiments involving 2-pack systems (discussed herein below).

In the practice of the present invention, the layer of applied curable fluid may form a continuous or discontinuous layer. The thickness of the applied layer of curable fluid may be uniform or it may vary. The amount of curable fluid that is applied to the substrate will depend on the substrates and on the use to which the composite article will be put. In some embodiments, a preferable amount of applied curable fluid is at least 0.32 $g/m^2$ (0.2 lb/ream); more preferable is at least 1.1 $g/m^2$ (0.7 lb/ream); still more preferable is at least 1.5 $g/m^2$ (0.9 lb/ream). Also, in some embodiments, a preferable amount of applied curable fluid is 4.5 $g/m^2$ (3 lb/ream) or less; more preferable is 2.1 $g/m^2$ (1.3 lb/ream) or less, and still more preferable is 1.8 $g/m^2$ (1.1 lb/ream) or less.

Those skilled in the art will recognize that the most desirable viscosity for the curable fluid will depend on the choice of coating method. For use with a roll coater, preferred viscosity is 1.0 Pa•s to 5.0 Pa•s (1,000 cps to 5,000 cps).

In some embodiments of the present invention, after the curable fluid is applied to a first substrate, it is contacted with a subsequent substrate to form an assembly, which is optionally subjected to applied pressure such as by passing it between rollers to effect increased contact of the substrates with the curable fluid. In another embodiment the curable fluid may be simultaneously or sequentially applied to two surfaces of a first substrate, which coated surfaces are then simultaneously or sequentially bonded to two subsequent substrates, which may be the same or different

relative to the first substrate and each other. It is further contemplated that the composite article may subsequently be bonded to one or more other substrates using the same or a different adhesive before or after the process described herein. Also, it is contemplated that a wide variety of arrangements of substrates and polymeric adhesive layers may be used to form the composite article. For example, multiple substrates may be alternated with multiple layers of adhesive, such as for example in multilayered laminates. For another example, in some embodiments, layers of curable fluid, each applied to its own substrate, may be brought together.

The substrates to be bonded in the method of this invention may be the same or different and include, for example, metal, wood, paper, elastomers, woven and nonwoven fabrics, and plastics which may have smooth or textured surfaces and are provided in the form of rolls, sheets, films, foils, *etc.* Suitable substrates include for example plywood, paper, impregnated paper, polystyrene foam, polyester film, polyester fabric, aluminum, steel, polyvinyl chloride, natural and synthetic rubber, polymer blends, and engineering plastics.

In some embodiments of the present invention, the substrates that are bonded are relatively thin and flat, and in such cases the composite article is called a laminate or laminated structure. Some flat substrates known to be suitable for the practice of the present invention include for example untreated polyethylene terephthalate (PET) films; PET films treated by corona discharge; PET films with chemically treated surface; polyolefin films, including low density polyethylene films, other polyethylene films, and polypropylene films; and metalized polymer films.

In embodiments of the present invention in which the curable fluid forms a polymeric adhesive composition used to bond substrates to each other, the solids level of the curable fluid is preferably 50% or higher; more preferably 75% or higher; even more preferably 95% or higher, and most preferably 99% or higher.

In some embodiments of the present invention, after the layer of curable fluid is applied to a first substrate, some or all of that layer of curable fluid kept out of contact with any subsequent substrate. After the curable fluid is exposed to cure conditions, the polymeric composition forms a coating on the substrate, and the combination of substrate and polymeric composition is a coated article. The substrate and the ingredients of the curable fluid are desirably chosen so that the coating will adhere well to the substrate and will give desirable properties to the surface, such as, for example, improved appearance and/or resistance to damage. In some of these coating embodiments, known as low solids embodiments, the solids level is sometimes desirably lower than the solids levels found in other embodiments. That is, as in other embodiments, the ingredients may be dissolved or dispersed in a solvent or a dispersing medium. However, it is contemplated that in the absence of subsequent substrates, the solvent and/or dispersing medium will more easily evaporate in a reasonable time during the time or soon after the time that the cure reactions take place. Thus, for low-solids coatings embodiments, suitable solids levels of the curable fluid are 10% or higher, based on the weight of the curable fluid; preferred are 20% or higher; more preferred is 40% or higher; still more preferred is 60% or higher. Also contemplated are high solids coatings embodiments, for which the solids level of the curable fluid is suitably 25% or higher; preferably 50% or higher; more preferably 75% or higher; even more preferably 95% or higher, and most preferably 99% or higher.

Photoinitiators are compounds that do not participate in polymerization but that form one or more radicals when exposed to radiation. Generally, photoinitiators are not needed in compositions that are cured by exposure to electron beam radiation. In the practice of the present invention, photoinitiators may be included in the curable fluid of the present invention. However, preferred curable fluids contain amounts of photoinitiator that are so low as to be ineffective; that is, any photoinitiator present is so dilute as to have no appreciable effect on the cured composite article; more preferred are curable fluids that contain no photoinitiator.

While the present invention is not limited to any particular mechanism or chemical reaction, it is contemplated that, upon exposure to cure condition, the polymerizable compounds in the curable fluid will polymerize. The polymerizable compounds may be monofunctional, multifunctional, or mixtures thereof, so that the polymers formed may be linear, branched, comb-structured, star-structured, and/or crosslinked. All such types of polymers are contemplated for use in the practice of the present invention. As used herein, "polymerization" refers to the chemical reaction of monomer molecules to form polymers; when multifunctional monomers are included, the polymerization process may also be referred to as "crosslinking." Polymerization, with or without crosslinking, is often referred to as "curing." In the practice of the present invention, it is contemplated that the curable fluid, upon exposure to cure condition, may undergo any or all of polymerization, curing, and/or crosslinking.

In the practice of the present invention, upon exposure to cure conditions, the curable moieties (*i.e.,* polymerizable compounds and/or curable PNPs) in the curable fluid undergo chemical reactions with each other and/or with other ingredients of the curable fluid to form a polymeric composition. In some embodiments, these chemical reactions will be among similar and/or identical functional groups; such embodiments are known herein as homogeneous-cure embodiments. For example, all the functional groups on the PNPs and on any polymerizable compounds in the curable fluid could be carbon-carbon double bonds, which could react with each other under cure conditions to form the polymeric composition. Other reactive groups suitable for use in homogeneous-cure embodiments include, for example, epoxide groups and isocyanate groups.

It is contemplated that some homogeneous cure embodiments will require that the curable fluid be exposed

to atmospheric ingredients such as air and/or water for the curing reaction to take place; in practicing such embodiments, it is contemplated that exposure to such atmospheric ingredients will be accomplished as needed, for example by direct application (for example, by spraying) or by indirect application (for example by diffusion through porous substrate). Such embodiments are considered homogeneous because the reactive groups in the curable fluid itself are the same as or similar to each other. In some embodiments, the curable fluid will be stored away from contact with the atmosphere, to inhibit the curing reaction, until the layer or curable fluid is applied to substrate.

In some embodiments of the present invention, these chemical reactions that take place under the cure conditions are between and/or among two or more complementary reactive groups. In some embodiments, some moieties in the curable fluid would have A functional groups and other moieties would have B functional groups, where A and B are complementary; such embodiments are known herein as heterogeneous-cure embodiments. The A groups and the B groups, independently, could be attached to some or all of the PNPs and to some or all of any polymerizable compounds in the curable fluid, as long as enough A and B groups are provided so that a polymeric composition is formed upon exposure of the curable fluid to cure condition. As examples, some embodiments include: A-functional PNPs with B-functional polymerizable compounds; A-functional PNPs with B-functional PNPs and polymerizable compounds having A and/or B functional groups. Any of the pairs of complementary functional groups discussed herein above are suitable as the A/B pairs for use in heterogeneous cure embodiments of the present invention. Either member of any complementary pair may be selected to function as either the A functional group or the B functional group, while the other member of the complementary pair will be selected to function as the B functional group or the A functional group, respectively.

In the practice of the present invention, the curable fluid may be a one-pack system or a multi-pack system. A one-pack system is a curable fluid in which the ingredients are all admixed together and then stored for long times, while the curable fluid remains useful in the practice of the present invention. That is, the one-pack system may be stored, without losing either its curable fluidity or is capability of forming a polymeric composition, for 1 week or more; preferably for 1 month or more; more preferably for 1 year or more; most preferably for 2 years or more. Embodiments of the present invention that use a one-pack system for the curable fluid are contemplated to include homogeneous-cure embodiments, heterogeneous embodiments, and embodiments in which the cure may be both heterogeneous and homogeneous. When heterogeneous embodiments using a one-pack system are practiced, the reactive groups will be chosen to be such that the cure reaction does not take place until the curable fluid is exposed to some cure condition other than merely making the admixture. Thus, embodiments using a one-pack system will have a cure condition that is one or more of elevated temperature, radiation, or a combination thereof; preferred are electron beam radiation, elevated temperature, or a combination thereof.

A multi-pack system is a combination of two or more precursor curable fluids that may be stored separately and then admixed with each other to form the curable fluid of the present invention a short time before the assembly is formed. While almost any embodiment of the present invention may be practiced using a 2-pack system, it is contemplated that the use of a 2-pack system will be most advantageous when heterogeneous-cure embodiments are practiced with reactive groups that react relatively quickly with each other. For example, one pack may contain one or more moieties with functionality A (as described herein above) while another pack may contain one or more moieties with complementary functionality B; if the reaction that takes place when the packs are admixed contributes in a useful way to the formation of the polymeric composition, then this admixture of moieties with reactive groups constitutes a cure condition of the present invention.

In the practice of 2-pack embodiments of the present invention, in many cases, as the groups react with each other, the viscosity of the curable fluid will rise. The reaction between groups should be long enough that the assembly can be assembled while the viscosity of the curable fluid is still below 10 Pa•s (10,000 cps); preferably, the viscosity will remain below 10 Pa•s (10,000 cps) for 10 s or longer; more preferably for 1 minute or longer; most preferably for 5 minutes or longer. Generally, when the reaction that takes place upon admixture of the packs contributes in a useful way to the formation of the polymeric composition, this contribution can be observed by measuring the rise in viscosity that takes place when the packs are admixed. Preferably, the admixture reaches viscosity of 50 Pa•s (50,000 cps) in 5 days or less; more preferably in 2 days or less; even more preferably in 1 day or less.

In some 2-pack embodiments, the admixing of the moieties with reactive groups will be a sufficient cure condition to form a useful polymeric composition. In other embodiments, additional cure conditions of elevated temperature and/or radiation will be used. For embodiments using 2-pack systems, preferred cure condition is a combination of mixing reactive groups and radiation; more preferred is a combination of mixing reactive groups and electron beam radiation.

In the practice of the present invention, the assembly is subjected to one or more polymerization conditions. In some embodiments, these conditions cause some or all of the curable fluid to cure (*i.e.,* to react chemically to become a polymer). Suitable minimum extent of cure is an extent sufficient to create a bond strength that is useful and to reduce the amount of non-polymerized material to acceptable levels. In embodiments that use monomer as an ingredient in the curable fluid, one measure of extent of polymerization is the percent conversion of monomer to polymer (PCMP),

which is defined herein as

$$PCMP = 100*(1 - (URM/TM))$$

where URM is the weight of monomer that is unreacted after the formation of the composite article, and TM is the weight of all monomer present in the curable fluid. Preferred extents of polymerization are 10% or greater; more preferred are 50% or greater; still more preferred is 75% or greater; even still more preferred is 90% or greater, and most preferred is 95% or greater.

In the practice of the present invention, the curable fluid is subjected to one or more cure conditions. In some embodiments, these conditions cause some or all of the curable fluid to cure (*i.e.*, to react chemically to become a polymer). Suitable minimum extent of cure conditions is an extent sufficient to create a bond strength that is useful and to reduce the amount of non-polymerized material to acceptable levels. One measure of extent of polymerization is the conversion of monomer to polymer, defined herein as the weight of monomer in the curable fluid that is chemically bound to a polymeric species or to substrate in the composite article, as a percentage of the weight of monomer in the curable fluid. Preferred extent of polymerizations are 10% or greater; more preferred are 50% or greater; still more preferred is 75% or greater; even still more preferred is 90% or greater, and most preferred is 95% or greater. The preferred cure conditions are elevated temperature, ultraviolet (UV) radiation; electron beam radiation, and mixtures thereof.

When elevated temperature is used as all or part of the cure condition of the present invention, suitable temperatures are 70°C or higher; preferred is 80°C or higher. Also preferred are temperatures low enough to avoid any substantial degradation of the composite article; preferred is 200°C or lower; more preferred is 150°C or lower; even more preferred is 100°C or lower. The elevated temperature is maintained for duration long enough to achieve the desired properties of the composite article; duration of elevated temperature generally will be shorter if higher temperatures are used. Preferred durations are 5 minutes or longer; more preferred is 30 minutes or longer; even more preferred is 1 hour or longer. Suitable durations are also 2 days or less; preferred is 1 day or less; more preferred is 10 hours or less. One combination of temperature and duration believed to be suitable is, for example, 80°C for 6 hours. When elevated temperature is used, a thermal initiator (*i.e.*, a compound that, when heated, forms one or more moieties that can initiate polymerization) is generally included in the curable fluid. Suitable initiators include, for example, 2,2'-azobis-(2-methylbutyronitrile).

When UV radiation is used as all or part of the cure condition of the present invention, a photoinitiator is generally included in the curable fluid. One photoinitiator believed to be suitable is, for example, 2-hydroxy-2-methyl-1-phenyl-propan-1-one, available as Darocure™ 1173 from Ciba Specialty Chemicals Inc. A preferred dose range of UV radiation is 50 mJ/cm$^2$ to 5,000 mJ/cm$^2$; more preferred is 200 mJ/cm$^2$ to 2,000 mJ/cm$^2$.

When electron beam radiation is used as all or part of the cure condition of the present invention, the dose of radiation is measured in SI units called "gray," abbreviated Gy, equivalent to 1 Joule of energy per kilogram of irradiated material, as described in "An Introduction to Radiation Units and Measurement," by H.C. Biggin, in Irradiation Effects on Polymers, edited by DW Clegg *et.al.,* Elsevier, 1991. One thousand gray units is one kilogray, or kGy. Another dosage unit is the rad, equivalent to 100 erg/gram; 1 Gy = 100 rad. In some embodiments, 5 kGy, (0.5 Megarad, abbreviated Mrad) or greater is suitable; preferred is 10 kGy (1 Mrad) or greater; more preferred is 20 kGy (2 Mrad) or greater. Higher energy levels may be used, but they add extra expense and slow down the speed of production of bonded composite. In some embodiments, 200 kGy (20 Mrad) or less is suitable; preferred is 100 kGy (10 Mrad) or less; more preferred is 50 kGy (5 Mrad) or less.

In the practice of the present invention it is contemplated that when radiation is used as a cure condition, the materials may become warmer than room temperature due to hot application of the curable fluid, to exothermic chemical reactions during cure, to conversion of radiation to heat, and/or to other causes.

For embodiments in which mixing of moieties with complementary reactive groups is used as all or part of the cure condition of the present invention, it is contemplated that the 2-pack systems would be commonly used. In such embodiments, the ingredients would be chosen so that the reaction of the complementary reactive groups was slow enough to allow the curable fluid to be applied to a substrate before its viscosity became too high. In some embodiments, the application of the curable fluid to a substrate would be performed at low temperature, which, it is believed, would slow the chemical reaction. In embodiments in which mixing of moieties with complementary reactive groups is the primary or only cure condition, the ingredients would be chosen so that the reaction of the complementary reactive groups was also fast enough to allow the composite article had a useful adhesive strength within a desirably short time. A useful embodiment would combine mixing of complementary reactive groups with other types of cure condition, such as elevated temperature (including possibly coating at low temperature and then curing at high temperature) and/or radiation. It is contemplated that when mixing of moieties with complementary reactive groups is used as a cure condition, the materials may become warmer than room temperature due to hot application of the curable

fluid, to exothermic chemical reactions during cure, to conversion of radiation to heat, and/or to other causes.

In some embodiments of the present invention, the curable fluid is a one-pack system. That is, either it employs homogeneous cure or it employs complementary reactive groups that do not react upon storage but only upon exposure to elevated temperature and/or radiation. This curable fluid is applied to a substrate; the curable fluid is then optionally contacted with a second substrate; and the fluid is then exposed to elevated temperature, radiation, or both.

In some embodiments of the present invention, the curable fluid is a two-pack system. The ingredients of each pack are chosen so that when the packs are mixed together, the curable fluid thus formed remains a fluid for more than 30 seconds but less than 24 hours. While the curable fluid remains a fluid, it is applied to a substrate. Subsequent substrates are optionally contacted with the curable fluid, and the fluid is then exposed to elevated temperature, radiation, or both.

In some embodiments of the present invention, the curable fluid contains curable PNPs but no other reactive, curable, or polymerizable ingredients.

In some embodiments of the present invention, the curable fluid contains non-curable PNPs and other ingredients that are not PNPs but that include polymerizable compounds.

In some embodiments of the present invention, the curable fluid contains curable PNPs, other ingredients that are not PNPs but that include polymerizable compounds.

It is to be understood that for purposes of the present specification and claims that the range and ratio limits recited herein can be combined. For example, if ranges of 60 to 120 and 80 to 110 are recited for a particular parameter, it is understood that the ranges of 60 to 110 and 80 to 120 are also contemplated. Additionally, if minimum range values of 1 and 2 are recited, and if maximum range values of 3, 4, and 5 are recited, then the following ranges are all contemplated: 1 to 3, 1 to 4, 1 to 5, 2 to 3, 2 to 4, and 2 to 5.

In the following Examples, these terms and abbreviations are used:

| | |
|---|---|
| AA = | acrylic acid |
| MMA = | methyl methacrylate |
| TMPTA = | trimethylolpropane triacrylate |
| HEMA = | 2-hydroxyethyl methacrylate |
| BA = | n-butyl acrylate |
| MorCure™ 2000 = | epoxy diacrylate, from Rohm and Haas Co. |
| TPGDA = | tripropylene glycol diacrylate |
| Ebecryl™ 524 = | acid-terminated polyester resin, from UCB Co. |
| Ebecryl ™ CL-1039 = | urethane monoacrylate from UCB Co. |
| parts = | parts by weight |
| OPP = | oriented polypropylene |
| EB = | electron beam |
| mil = | 25.4 µm (0.001 inch) |

EXAMPLES

Example 1 Preparation of PNPs

A 500 mL reactor is fitted with a thermocouple, a temperature controller, a purge gas inlet, a water-cooled reflux condenser with purge gas outlet, a stirrer, and an addition funnel. To the addition funnel is charged 201.60 g of a monomer mixture consisting of 18.00 g methyl methacrylate (100% purity), 2.00 g diethyleneglycol dimethacrylate (100% purity), 1.60 g of a 75% solution of t-amyl peroxypivalate in mineral spirits (Luperox™ 554-M-75), and 180.00 g diisobutyl ketone ("DIBK"). The reactor, containing 180.00 g DIBK is then flushed with nitrogen for 30 minutes before applying heat to bring the contents of the reactor to 75° C. When the contents of the reactor reaches 75° C, the monomer mixture in the addition funnel is uniformly charged to the reactor over 90 minutes. Thirty minutes after the end of the monomer mixture addition, the first of two chaser aliquots, spaced thirty minutes apart and consisting of 0.06 g of a 75% solution of t-amyl peroxypivalate in mineral spirits (Luperox™ 554-M-75) and 2.00 g DIBK, is added. At the end of the second chaser aliquot, the contents of the reactor is held $2^1/_2$ hours at 80° C to complete the reaction. The resulting polymer is isolated by precipitation with heptane, collected by filtration and dried under vacuum to yield a white powder. This material is redissolved in propyleneglycol monomethylether acetate. The PNPs thus formed have a mean particle size of between 1 nm and 50 nm as determined by dynamic light scattering.

Example 2

Polymeric nanoparticles of mean particle diameter between 1 nm and 50 nm with pendant acrylic unsaturation

(4 reactive sites/molecule) are prepared by making PNPs based on MMA/TMPTA/HEMA, using the methods of Example 1, which are then functionalized by reaction with AA.

Example 3

Polymeric nanoparticles of mean particle diameter between 1 nm and 50 nm with pendant acrylic unsaturation (4 reactive sites/molecule) are prepared by making PNPs based on BA/TMPTA/HEMA, using the methods of Example 1, which are then functionalized by reaction with AA.

Examples 4-7

Examples 4-7 are formulated as follows:

| Ingredient | Example 4 | Example 5 | Example 6 | Example 7 |
|---|---|---|---|---|
| Example 2 | 30 parts | 30 parts | | |
| MorCure 2000 | 20 parts | 30 parts | | |
| TPGDA | 50 parts | 40 parts | | |
| Example 3 | | | 30 parts | 40 parts |
| Ebecryl™ 524 | | | 40 parts | 40 parts |
| CL-1039 | | | 30 parts | 20 parts |

Each of Examples 4-7 has viscosity of 10 Pa•s (10,000 cps) or less at 60°C.

Example 8

The compositions of Examples 4 and 5 are each coated at 2.5 µm (0.1 mil) thickness onto 25 µm (1 mil) OPP film, exposed to 30 kGy (3 Mrad) of EB radiation and result in cured, hard film coatings.

Example 8

The compositions of Examples 6 and 7 are coated at 2.5 µm (0.1 mil) onto 25 µm (1 mil) OPP film and mated with a second film of the same, exposed to 30 kGy (3 Mrad) of EB radiation and result in cured, flexible adhesives yielding adhesion to both OPP films.

## Claims

1. A curable fluid comprising polymeric nanoparticles, wherein said curable fluid is capable of forming a polymeric composition after exposure to at least one cure condition selected from the group consisting of elevated temperature, radiation, mixing of moieties with complementary reactive groups, and combinations thereof; wherein said polymeric nanoparticles comprise, as polymerized units, at least one multiethylenically unsaturated monomer; and wherein said polymeric nanoparticles have mean diameter of 1 nm to 50 nm.

2. The fluid of claim 1, wherein said cure condition comprises electron beam radiation.

3. The fluid of claim 1, wherein said polymeric nanoparticles are made by solution polymerization.

4. The fluid of claim 1, wherein said polymeric nanoparticles have mean diameter of 1 nm to 20 nm.

5. The fluid of claim 1, wherein said cure condition comprises electron beam radiation, wherein said polymeric nanoparticles are made by solution polymerization, and wherein said polymeric nanoparticles have mean diameter of 1 nm to 20 nm.

**6.** A composite article comprising a first substrate, a polymeric adhesive composition, and at least one subsequent substrate, wherein said composite article is formed by a process comprising:

(a) applying a layer of a curable fluid comprising polymeric nanoparticles, wherein said polymeric nanoparticles comprise, as polymerized units, at least one multiethylenically unsaturated monomer, and wherein said polymeric nanoparticles have mean diameter of 1 nm to 50 nm,
(b) contacting said layer to said subsequent substrate, and
(c) exposing said curable fluid to at least one cure condition selected from the group consisting of elevated temperature, radiation, mixing of moieties with complementary reactive groups, and combinations thereof.

**7.** The composite article of claim 6, wherein said cure condition comprises electron beam radiation, wherein said polymeric nanoparticles are made by solution polymerization, and wherein said polymeric nanoparticles have mean diameter of 1 nm to 20 nm.

**8.** A method for forming a composite article comprising

(a) applying a layer of a curable fluid comprising polymeric nanoparticles to a first substrate, wherein said polymeric nanoparticles comprise, as polymerized units, at least one multiethylenically unsaturated monomer, and wherein said polymeric nanoparticles have mean diameter of 1 nm to 50 nm,
(b) contacting said layer to at least one subsequent substrate, and
(c) exposing said curable fluid to at least one cure condition selected from the group consisting of elevated temperature, radiation, mixing of moieties with complementary reactive groups, and combinations thereof;

wherein, subsequent to exposure of said curable fluid to said cure condition, a polymeric adhesive composition has formed.

**9.** The method of claim 8, wherein said cure condition comprises electron beam radiation, wherein said polymeric nanoparticles are made by solution polymerization, and wherein said polymeric nanoparticles have mean diameter of 1 nm to 20 nm.

**10.** A coated article comprising a substrate and a polymeric coating composition, wherein said coated article is formed by a process comprising:

(a) applying a layer of a curable fluid comprising polymeric nanoparticles to said substrate, wherein said polymeric nanoparticles comprise, as polymerized units, at least one multiethylenically unsaturated monomer, and wherein said polymeric nanoparticles have mean diameter of 1 nm to 50 nm, and
(b) exposing said curable fluid to at least one cure condition selected from the group consisting of elevated temperature, radiation, mixing of moieties with complementary reactive groups, and combinations thereof.

**11.** The composite article of claim 10, wherein said cure condition comprises electron beam radiation, wherein said polymeric nanoparticles are made by solution polymerization, and wherein said polymeric nanoparticles have mean diameter of 1 nm to 20 nm.

**12.** A method for forming a coated article comprising

(a) applying a layer of a curable fluid comprising polymeric nanoparticles to a substrate, wherein said polymeric nanoparticles comprise, as polymerized units, at least one multiethylenically unsaturated monomer, and wherein said polymeric nanoparticles have mean diameter of 1 nm to 50 nm, and
(b) exposing said curable fluid to at least one cure condition selected from the group consisting of elevated temperature, radiation, mixing of moieties with complementary reactive groups, and combinations thereof;

wherein, subsequent to exposure of said curable fluid to said cure condition, a polymeric coating composition has formed.

**13.** The method of claim 12, wherein said cure condition comprises electron beam radiation; wherein said polymeric nanoparticles are made by solution polymerization; wherein said polymeric nanoparticles have mean diameter of 1 nm to 20 nm.